# EUROPEAN PATENT APPLICATION

(11) **EP 3 552 540 A1**
(43) Date of publication of application: **16.10.2019**
(21) Application number: 19168185.7
(22) Date of filing: 09.04.2019
(51) Int. Cl.: A61B 5/00, A61B 5/042, A61B 5/06

(54) **CATHETER LOCALIZATION USING FIBER OPTIC SHAPE SENSING COMBINED WITH CURRENT LOCATION**

(30) Priority: 10.04.2018 US 201815949217
(71) Applicant: Biosense Webster (Israel) Ltd., Yokneam, 2066717 (IL)
(72) Inventor: LUDWIN, Doron Moshe, 3220732 Haifa (IL); COHN, Goren, 2066717 Yokneam (IL); YELLIN, Tamir Avraham, 2066717 Yokneam (IL); FLEISHON, Gal, 3269506 Haifa (IL); SCHECHTER, Menachem, 2066717 Yokneam (IL)
(74) Representative: Small, Gary James

(57) **Abstract**

An apparatus includes a shaft, a flexible distal-end assembly, two or more sensing-electrodes, and a fiber-optic shape sensor. The shaft is configured for insertion into a body of a patient. The flexible distal-end assembly is fitted at a distal end of the shaft. The two or more sensing-electrodes are disposed over the distal-end assembly and are configured to generate signals indicative of positions of the sensing-electrodes in the body. The fiber-optic shape sensor is coupled to a portion of the distal-end assembly, wherein the two or more sensing-electrodes have α-priori known distances from a known location over the fiber-optic shape sensor, and wherein the fiber-optic shape sensor is configured to provide an indication of a spatial deformation of the flexible distal-end assembly.

## Description

### FIELD OF THE INVENTION

The present invention relates generally to sensing a position of an object placed within a living body, and specifically to providing an accurate reference for impedance-based position sensors.

### BACKGROUND OF THE INVENTION

Tracking the position of intrabody objects, such as insertion tubes, catheters and implants, is required in many medical procedures. For example, U.S. Patent Application Publication 2011/0319910 describes a method, system, and apparatus for sensing or measuring the shape or position and shape of one or more parts of a shapeable elongate medical instrument for minimally-invasive intervention or diagnosis using shape data. The method, which includes obtaining a plurality of localized shape data, comprises using an impedance based localization system and where the shapeable instrument includes at least one sensor, where the system further includes at least one electrode where the impedance based localization system determines a voltage gradient between the sensor and the electrode.

As another example, U.S. Patent Application Publication 2008/0218770 describes a medical instrument assembly configured to maneuver an elongate medical instrument, includes a first member coupled to a second member by a movable joint, with a fiber sensor coupled to the first and second members, such that relative movement of the first and second members about the movable joint causes a bending of at least a portion of the fiber sensor. The fiber sensor has a proximal end operatively coupled to a controller configured to receive signals from the fiber sensor indicative of a bending thereof, the controller configured to analyze the signals to determine relative positions of the first and second members about the movable joint.

U.S. Patent Application Publication 2014/0095105 describes an algorithm to correct and/or scale an electrical current-based coordinate system that can include the determination of one or more global transformation or interpolation functions and/or one or more local transformation functions. The global and local transformation functions can be determined by calculating a global metric tensor and a number of local metric tensors. The metric tensors can be calculated based on predetermined and measured distances between closely-spaced sensors on a catheter.

U.S. Patent Application Publication 2008/0190438 describes methods and systems for determining information about a position of an object within a distribution of materials having different complex conductivities. The method includes: (i) causing current to flow in the distribution; (ii) measuring an electrical signal at each of multiple locations in the distribution of materials in response to the current flow; (iii) providing spatial information about the distribution of materials with respect to a first reference frame, the spatial information indicative of regions of different complex conductivity in the distribution of materials; and (iv) determining the position of the object with respect to the spatial information about the distribution of materials based on measured electrical signals and the spatial information. In an embodiment, the object is a catheter inserted into a patient's heart cavity for cardiac mapping.

### SUMMARY OF THE INVENTION

An embodiment of the present invention provides an apparatus including a shaft, a flexible distal-end assembly, two or more sensing-electrodes, and a fiber-optic shape sensor. The shaft is configured for insertion into a body of a patient. The flexible distal-end assembly is fitted at a distal end of the shaft. The two or more sensing-electrodes are disposed over the distal-end assembly and are configured to generate signals indicative of positions of the sensing-electrodes in the body. The fiber-optic shape sensor is coupled to a portion of the distal-end assembly, wherein the two or more sensing-electrodes have a-priori known distances from a known location over the fiber-optic shape sensor, and wherein the fiber-optic shape sensor is configured to provide an indication of a spatial deformation of the flexible distal-end assembly.

In some embodiments, the apparatus further includes a processor configured to estimate a position of the distal-end assembly in the body based on (i) the signals generated by the sensing-electrodes, (ii) the a-priori known distances and (iii) the indication of the spatial deformation provided by the fiber-optic shape sensor.

In some embodiments, the processor is configured to estimate the position of the distal-end assembly by estimating position coordinates of the sensing-electrodes using the generated signals, by locally-scaling the position coordinates based on the a-priori known distances, and by correcting the locally-scaled position coordinates based on the indication of the spatial deformation provided by the fiber-optic shape sensor.

There is additionally provided, in accordance with an embodiment of the present invention, a method for position sensing, the method including inserting a shaft into a body of a patient, wherein a flexible distal-end assembly is fitted at a distal end of the shaft, wherein two or more sensing-electrodes are disposed on the distal-end assembly and are configured to generate signals indicative of positions of the sensing-electrodes in the body, wherein a fiber-optic shape sensor is coupled to a portion of the distal-end assembly, wherein the two or more sensing-electrodes have a-priori known distances from a known location over the fiber-optic shape sensor, and wherein the fiber-optic shape sensor is configured to provide an indication of a spatial deformation of the flexible distal-end assembly. Position coordinates of the two or more sensing-electrodes in the body cavity are measured using the generated signals, and using the indication of the spatial deformation provided by the fiber-optic shape sensor.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a position tracking system, in accordance with an embodiment of the present invention;
Fig. 2 is a schematic detail view showing a flexible lasso catheter comprising a fiber optic shape sensor and multiple sensing-electrodes, in accordance with an embodiment of the present invention;
Fig. 3 is a is a schematic illustration of the flexible lasso catheter of Fig. 2, in straight and deformed states, in accordance with an embodiment of the present invention; and
Fig. 4 is a flow chart that schematically illustrates a method for accurately mapping a cavity in the body, in accordance with an embodiment of the present invention.

### DETAILED DESCRIPTION OF EMBODIMENTS

### OVERVIEW

Some medical procedures require accurate spatial mapping of an anatomy of a patient, such as that of a left atrium of a heart. Embodiments of the present invention that are described herein provide position sensing systems and methods, where a Fiber-Optic Shape-Sensing sensor (FOSS sensor) is coupled to a flexible distal end assembly of a medical instrument (e.g., catheter) that performs spatial mapping. The FOSS sensor provides an indication of the spatial deformation of the flexible distal end assembly during spatial mapping. This indication is used for correcting electrical impedance-based measurements of positions of the flexible distal end assembly, which initially assume that the distal end assembly is not deformed.

The position of a distal end of a catheter can be estimated by measuring impedances between sensing-electrodes fitted at the distal end of the catheter and surface electrodes attached to the patient's skin. In principle, the impedance-based technique is sufficient for deriving a position of a sensing-electrode, for example in a heart. In practice, however, the resulting position accuracy is insufficient.

In the description hereinafter, a Pure Active Current Location (PureACL) impedance-based system and technique, made by Biosense-Webster, Inc., serves as an example of impedance-based position tracking systems, while a catheter using such sensing-electrodes is named 'PureACL catheter'. The surface electrodes are named hereinafter 'ACL patches'. In some embodiments, in order to improve the positioning accuracy, a calibration catheter is first inserted into the heart. The calibration catheter comprises a magnetic position sensor, and sensing-electrodes similar to those of the PureACL catheter. The calibration catheter is used for producing a calibration map, in which accurate position measurements by the magnetic sensor are correlated with less accurate PureACL (impedance-based) measurements.

A PureACL catheter that is subsequently inserted into the heart uses the calibration map to provide the physician a correct position of its distal end in the heart, using only sensing-electrodes (i.e. using PureACL impedance-based method).

In many practical cases, the accuracy of magnetically calibrated PureACL position sensing can be further improved using a 'local scaling' process. In some embodiments, such a process, named hereinafter Independent Current Location' (ICL), is applied so as to further improve the accuracy of magnetically calibrated PureACL positions. The ICL process is applicable to catheters having a plurality of sensing-electrodes disposed over their distal end. Using one or more known distances between neighboring electrodes, among other inputs, the ICL process is able to scale the relative positions of a plurality of electrodes as to exactly fit the shape of the distal end of the PureACL catheter, finally providing highly accurate electrode positions. The assumption used in ICL, that distances between neighboring electrodes are always known, is valid as long as the rigidity of a distal end of a catheter is sufficient to withstand very local deformations. If this assumption is not valid, i.e., if the catheter distal end deforms by more than a permitted amount, the local scaling process does not provide the expected accuracy.

In practice, the shape of a flexible distal end assembly does deform during mapping, but on a scale of the entire assembly. Since the magnetically calibrated PureACL and ICL methods can provide accurate positions only when the distal end assembly is un-deformed, the deformation causes errors in the sensing-electrode positions derived by the magnetically calibrated PureACL and ICL method.

In some embodiments of the present invention, a FOSS sensor is coupled to a flexible distal end assembly. A known position over the fiber, such as a location at a given length from the fiber optic distal end (i.e., distal-most edge of the fiber), is used as a reference point to calculate a relative deformation of the flexible distal end assembly. Whenever the flexible distal end assembly is deformed, the FOSS sensor provides an indication of the deformation. The indication is used for correcting the magnetically calibrated electrical impedance-based derived electrode positions.

In this description, the terms 'flexible PureACL catheter', 'flexible distal-end assembly' and 'flexible catheter', are used interchangeably.

In some embodiments, a flexible PureACL lasso catheter is provided, comprising a flexible base segment section and a spiral end section. A FOSS sensor is coupled to the flexible PureACL lasso catheter. Sensing-electrodes may be distributed over the base segment section and/or over the spiral end section. When part of the lasso catheter, such as the base segment section, is deformed during a mapping procedure, actual positions of sensing-electrodes are displaced relative to the positions derived using PureACL and ICL (e.g. due to bending, deflection and/or twist of the flexible base segment section). The indication of the deformation that the FOSS sensor provides yields correctly derived positions of the sensing-electrodes.

FOSS sensors are typically based on optical fibers. Shape sensing using optical fibers exploits the strain sensitivity of light propagating in an optical fiber waveguide core. When such a core is offset from the center of a fiber it experiences a strain that depends on the curvature of the fiber. With more than one offset core, the direction of the bend may also be determined. Various techniques exist that extract a fiber shape from fiber-guided optical signals, and any such technique can be used for implementing the disclosed medical instruments.

The disclosed systems and methods provide highly accurate spatial and electrophysiological mapping capabilities. These capabilities are obtained by combining compact fiber-sensor direction sensing with the relatively low cost and simplicity that characterize the PureACL and ICL impedance-based position sensing. Moreover, the inherent compactness of the FOSS sensor opens the way for building compact and flexible sensing and/or ablating catheters.

### SYSTEM DESCRIPTION

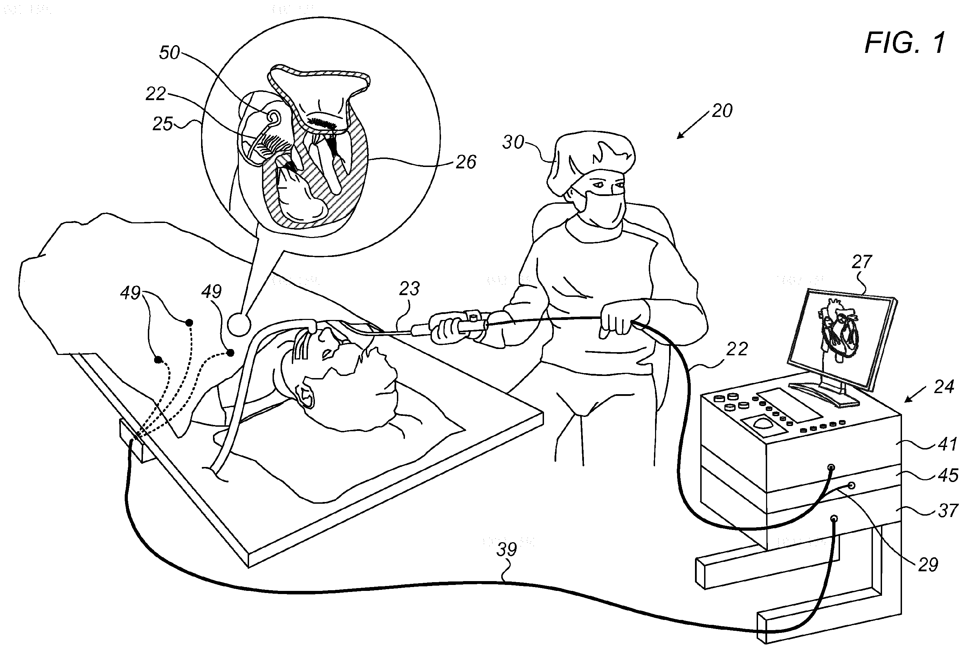

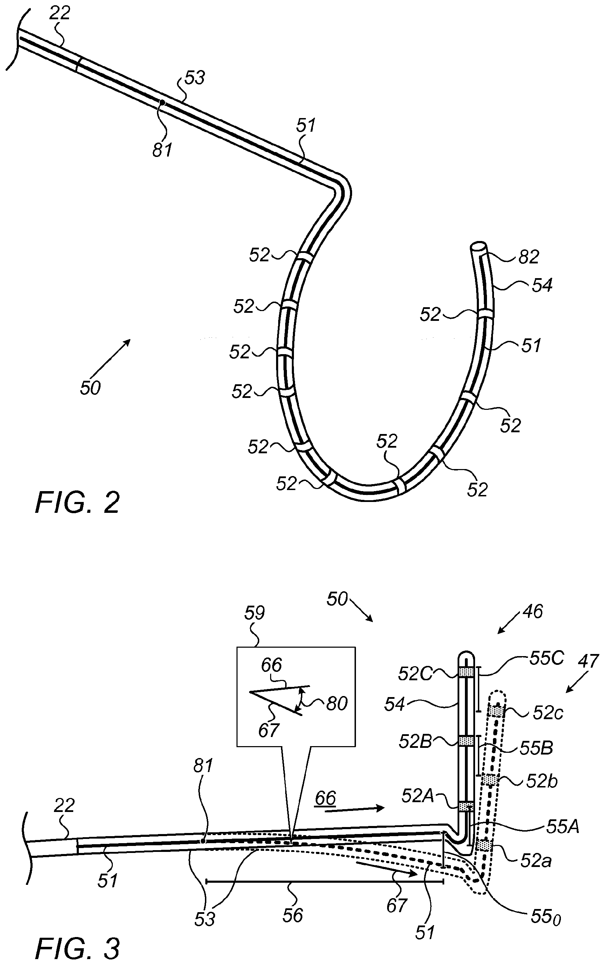

Fig. 1 is a schematic, pictorial illustration of a position tracking system 20, in accordance with an embodiment of the present invention. System 20 is used in determining the position of a flexible PureACL catheter, such as a flexible lasso catheter 50, seen in an inset 25 fitted at a distal end of a shaft 22. As explained above, PureACL catheter 50 incorporates sensing-electrodes (shown in Fig. 2) similar to those of a PureACL calibrating catheter, but need not include magnetic field sensors.

Lasso catheter 50 is inserted while being folded through a sheath 23 into an internal body cavity, such as a chamber of a heart 26 of a patient 28. By containing lasso catheter 50 in a folded configuration, sheath 23 also serves to minimize vascular trauma along the way to target location. Physician 30 navigates lasso catheter 50 to a target location in heart 26 by manipulating shaft 22 using a manipulator near the proximal end of the catheter and/or deflection from the sheath 23. After sheath 23 is retracted Lasso catheter 50 regains its intended functional shape.

Typically, lasso catheter 50 is used for diagnostic or therapeutic treatment, such as spatially mapping the heart, and mapping respective electrical potentials in the heart prior to performing an ablation of heart tissue. Other types of catheters or other intrabody devices may alternatively be used with system 20 for other purposes, by themselves or in conjunction with other treatment devices, such as ablating catheters.

As noted above, lasso catheter 50 comprises multiple sensing-electrodes. These sensing-electrodes are connected by wires running through shaft 22 to driver circuitry in a console 24. Console 24 comprises a processor 41, typically a general-purpose computer, with suitable front end and interface circuits 37 for receiving signals from PureACL patches 49. Processor 41 is connected to PureACL patches 49, which are attached to the chest skin of patient 26, by wires running through a cable 39.

In some embodiments, processor 41 accurately determines position coordinates of the sensing-electrodes fitted at lasso catheter 50 inside heart 26. Processor 41 determines the position coordinates based on, among other inputs, measured impedances between the sensing-electrodes (on the catheter) and ACL patches 49 (i.e., using PureACL and ICL methods described above). Console 24 drives a display 27, which shows the distal end of catheter position inside the body.

The method of electrode position sensing using system 20 is implemented in various medical applications, for example in the CARTO™ system, produced by Biosense Webster Inc. (Irvine, California) and is described in detail in U.S. Patents 7,756,576, 7,869,865, and 7,848,787, whose disclosures are all incorporated herein by reference.

Console 24 further comprises a FOSS unit 45, which typically comprises a light-source and a spectrometer, both coupled to one or more optical fibers. The one or more optical fibers run through shaft 22 and couple FOSS unit 45 with a FOSS sensor (which is included in catheter 50, as seen in Fig.2). As seen in Fig. 1, a cable 29 that includes the one or more optical fibers splits off shaft 22 to enter console 24 and connect to unit 45. FOSS unit 45 transmits an optical signal to the FOSS sensor, and receives a return optical signal from the FOSS sensor, indicative of a spatial deformation of catheter 50. FOSS unit 45 analyzes the return optical signal and provides a corresponding electrical input to processor 41, which uses it to calculate a displacement of the sensing-electrodes due to the deformation of catheter 50, as to correct the sensing-electrodes PureACL and ICL derived positions. FOSS unit 45 and the FOSS sensor are further described below.

In some embodiments, processor 41 is further configured to estimate a position of lasso catheter 50 (i.e., of the flexible distal-end assembly of catheter 50) in the body based on (i) signals generated by the sensing-electrodes (ii) the *a-priori* known distances between the sensing-electrodes and a known position over the fiber-optic, and (iii) indication of a spatial deformation provided by a fiber-optic shape sensor (seen in Fig.2), as explained below.

Processor 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

The elements of system 20 and the methods described herein may be applied for position-sensing and/or controlling ablation using many sorts of multi-electrode catheters, such as balloon, basket, and multi-arm catheters. Impedance based measurements can also be performed by applying voltage gradient using ACL patch electrodes 49 or other skin attached electrodes, and measure the potential voltage with two or more of the sensing electrodes on catheter 50. (e.g., using the Carto®4 technology produced by Biosense Webster Inc. (Irvine, California)). Thus, embodiments of the present invention apply to any position sensing method in which sensing-electrodes generate signals indicative of their position in the body.

### CATHETER LOCALIZATION USING FOSS COMBINED WITH CURRENT LOCATION

Fig. 2 is a schematic detail view showing flexible lasso catheter 50 comprising a fiber optic shape sensor, (FOSS) 51, and multiple sensing-electrodes 52, in accordance with an embodiment of the present invention.

As seen in Fig. 2, lasso catheter 50 is fitted at the distal end of shaft 22. Flexible lasso catheter 50 comprises a flexible base segment 53 and lasso guidewire 54, to both which FOSS sensor 51 is coupled. As seen, FOSS sensor 51 has an ending 82, which is close to the distal edge of lasso guidewire 54. Sensing-electrodes 52 are circumferentially distributed over lasso guidewire 54. In the fully expanded state of catheter 50, lasso guidewire 54 lies in a plane normal to a longitudinal axis defined by the distal end of shaft 22. A known position 81 over the fiber, which is located at a given length from the fiber optic distal ending 82, serves as reference point for processor 41 to calculate a deformation of flexible lasso catheter 50 relative to, as described below.

The catheter configuration described in Fig. 2 is chosen purely for the sake of conceptual clarity. In reality, lasso guidewire 54 may comprise one or more windings about the longitudinal axis defined by the distal end of shaft 22, or less than a single winding. In alternative embodiments, other flexible catheters can be fitted at the distal end of shaft 22, such as a PENTARAY® mapping catheter that comprises multiple arms.

FOSS sensor 51 may comprise one or more optical fibers. Only a simplified fiber section is illustrated in the figure, where all other elements of FOSS sensor 51 are omitted for clarity. In some embodiments, FOSS sensor 51 may comprise one or more patterned optical-fibers that generate one or more return optical signals in response to an incident optical signal that FOSS unit 45 transmitted. The patterns on the fibers may be spatially encoded (e.g., comprise multiple gratings patterned along a fiber sensing section, which have a position-dependent periodicity). Hence, return optical signals can provide indication of both the measure of deformation and its location along the fiber.

FOSS unit 45 analyzes the return signals so as to provide an indication of one or more deformations (e.g., bending and/or deflection and/or twist) and their respective locations, which can be used to calculate a displacement correction for the positions of the two or more sensing-electrodes 52, as further explained below.

Example FOSS sub-systems that can be used for implementing sensors 51 and units 45 are described, for example, in U.S. Patent Application Publication 2006/0013523. Other FOSS techniques are described, by Gander et. al., in "Bend measurement using Bragg gratings in multi-core fibre," Electronics Letters, Volume 36, Issue 2, January, 2000, pages 120-121, and in U.S. Patent 7,772,541, to name just few examples.

Alternatively, any other suitable FOSS sensors can be used. FOSS techniques have been commercialized for medical applications, for example by companies such as OFS FITEL, LLC, USA, and by Luna Innovations Incorporated, USA.

Generally, other types of shape sensors, such as one composed from (electrical) strain gauges, may be coupled to base segment 53 of a flexible distal end assembly, so as to provide an indication of the deformation of base segment 53.

Fig. 3 is a is a schematic illustration of the flexible lasso catheter of Fig. 2, in straight and deformed states, in accordance with an embodiment of the present invention. Base segment 53 of lasso catheter 50 is seen in an un-deformed state 46, and also in a deformed state 47. When lasso base segment 53 is un-deformed, its un-deformed-direction 66 is parallel to that of the longitudinal axis defined by the distal end of shaft 22. When base segment 53 is deformed, base segment 53 (and with it lasso guidewire 54) points at a different direction, a deformed-direction 67.

As inset 59 of Fig. 3 shows the exemplified deformation of flexible base segment 53 (i.e., of the flexible distal-end assembly). The exemplified deformation is characterized by a deflection angle 80 at a location 81. FOSS unit 45 analyzes the optical signals that FOSS sensor 51 provides so as to derive an indication of angle 80. Another indication FOSS sensor provides is the location 81 along the fiber where the deflection occurs, as explained above. A length 56 is readily derived, based on the known length of base segment 53, from location 81 to the end of base segment 53 (e.g., by FOSS unit 45). Using angle 80 and length 56, processor 41 calculates a displacement 55₀.

As seen, the positions of sensing-electrodes 52A, 52B and 52C also change (approximately) by displacement 55₀, to positions 52a, 52b and 52c, respectively. Thus, displacement 55₀ serves as a good approximation of displacements 55A, 55B and 55C, which characterize the majority of position change that electrodes 52A, 52B and 52C experience as a result of the exemplified deformation. Using displacement 55₀ as an input, processor 41 can correct the less accurate position of the flexible distal-end assembly (derived by PureACL and ICL).

The specific type of deformation of base segment 53 shown in Fig. 3 is a deflection by an angle 80 within a plane. This deformation is depicted purely by way of example. In general, the deformation of base segment 53 of lasso catheter 50 may comprise any deformation in space, e.g., a combination of bending and/or deflection (relative to the longitudinal axis) and twist (about the longitudinal axis). FOSS sensor 51 is configured to provide an indication of the deformation of base segment 53. The indication is used by processor 41 to calculate a general displacement 55₀ in space. In some embodiment, based on indication from FOSS sensor 51, processor 41 calculates a set of n displacements, {55ₙ}, wherein each of an n number of electrodes 52 fitted at catheter 50 has a distinct displacement calculated for.

In an embodiment, lasso catheter 50 deforms within a certain plane in space (e.g., the deformation shown in Fig. 3). This example form of bending may occur, for example, due to the catheter structure (e.g., rigidity properties of base segment 53), or due to the nature of the anatomy being probed. In such cases the measurement of deformation is essentially one dimensional and a simplified FOSS sensor may be fitted and/or a simplified FOSS method be employed.

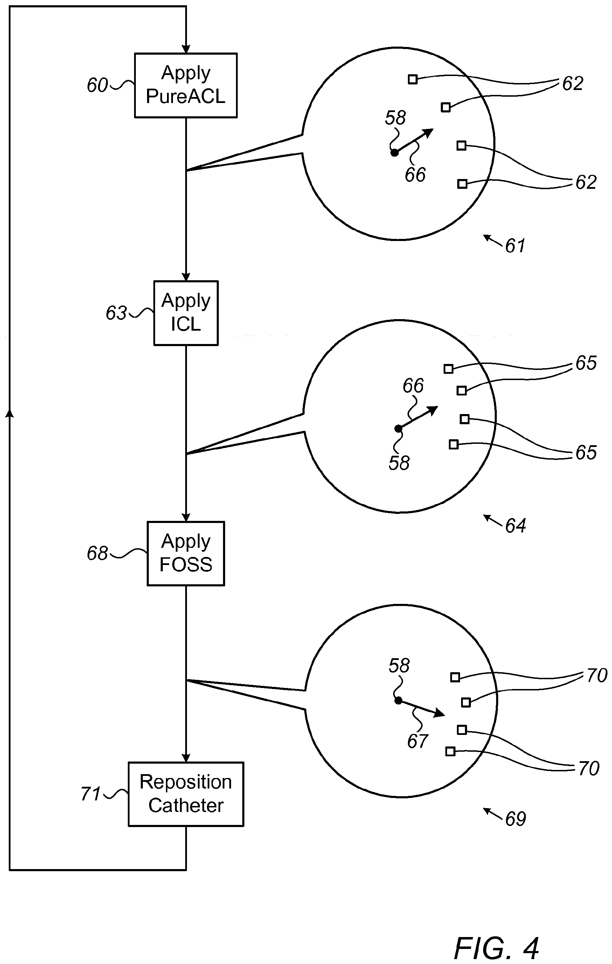

Fig. 4 is a flow chart that schematically illustrates a method for accurately mapping a cavity in the body, e.g., a cardiac chamber, in accordance with an embodiment of the present invention. At the start of the mapping procedure, processor 41 calculates positions 62 of sensing-electrodes 52, at a PureACL step 60. The positions are calculated at a given body location relative to an arbitrary origin 58, shown in inset 61. An un-deformed direction 66 of lasso catheter 50 is assumed (direction 66 seen in inset 61), and the resulting calculated electrode positions 62 are thus spread about un-deformed direction 66 relative to origin 58.

Next, processor 41 calculates a local scaling factor for electrodes positions using an ICL local scaling process, at an ICL step 63. The result is shown in an inset 64, where now derived positions 65 of sensing-electrodes are accurate relative to arbitrary origin 58.

Derived electrodes positions 65 are still inaccurate compared with their real positions, due to the deformation of the flexible distal-end assembly of lasso catheter 50 that was not taken into account. To correct this error, FOSS sensor 51 provides an indication of a spatial deformation of the flexible distal-end assembly, in the form of displacement 55₀, at a FOSS step 68, for example while catheter lasso 50 is moved within the volume to be mapped. Processor 41 corrects electrodes positions 65 by adding displacement 55₀. As seen in an inset 69, the resulting calculated electrode positions 70 are now correctly derived to spread about the FOSS measured deformed direction 67 relative to arbitrary origin 58.

As physician 30 moves lasso catheter to a new position in the cavity, at a repositioning step 71, the procedure repeats itself, looping back to ACL step 60, until physician 30 received the full mapping of a cavity, for example that of a left atrium of a heart.

The example flow chart shown in Fig. 4 is chosen purely for the sake of conceptual clarity. For example, in an embodiment, based on indication from FOSS sensor 51, processor 41 calculates two or more distinct (e.g., different in direction) displacements of respective two or more of electrodes 52. In alternative embodiments, the order of steps may change (e.g. ACL and FOSS steps may occur in parallel), and additional steps may be used, such as magnetic sensing of catheter position. The ICL method was scarcely presented, for sake of clarity. More algorithmic steps than presented are usually included in the ICL method. For example, the actual position may be determined by the ICL method by averaging the local scaling factors for each of the body voxels that the catheter has traversed nearby.

Although the embodiments described herein mainly address cardiac applications, the methods and systems described herein can also be used in other applications, such as in otolaryngology, neurology, sinuplasty, and pulmonary angioplasty.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. An apparatus, comprising:
a shaft for insertion into a body of a patient;
a flexible distal-end assembly, which is fitted at a distal end of the shaft;
two or more sensing-electrodes, which are disposed over the distal-end assembly and are configured to generate signals indicative of positions of the sensing-electrodes in the body; and
a fiber-optic shape sensor, which is coupled to a portion of the distal-end assembly, wherein the two or more sensing-electrodes have *a-priori* known distances from a known location over the fiber-optic shape sensor, and wherein the fiber-optic shape sensor is configured to provide an indication of a spatial deformation of the flexible distal-end assembly.

2. The apparatus according to claim 1, and comprising a processor configured to estimate a position of the distal-end assembly in the body based on (i) the signals generated by the sensing-electrodes, (ii) the *a-priori* known distances and (iii) the indication of the spatial deformation provided by the fiber-optic shape sensor.

3. The apparatus according to claim 2, wherein the processor is configured to estimate the position of the distal-end assembly by:
estimating position coordinates of the sensing-electrodes using the generated signals;
locally-scaling the position coordinates based on the *a-priori* known distances; and
correcting the locally-scaled position coordinates based on the indication of the spatial deformation provided by the fiber-optic shape sensor.

4. A method for position sensing, comprising:
inserting a shaft into a body of a patient, wherein a flexible distal-end assembly is fitted at a distal end of the shaft, wherein two or more sensing-electrodes are disposed on the distal-end assembly and are configured to generate signals indicative of positions of the sensing-electrodes in the body, wherein a fiber-optic shape sensor is coupled to a portion of the distal-end assembly, wherein the two or more sensing-electrodes have *a-priori* known distances from a known location over the fiber-optic shape sensor, and wherein the fiber-optic shape sensor is configured to provide an indication of a spatial deformation of the flexible distal-end assembly; and
measuring position coordinates of the two or more sensing-electrodes in the body cavity using the generated signals, and using the indication of the spatial deformation provided by the fiber-optic shape sensor.

5. The method according to claim 4, wherein measuring the position coordinates comprises receiving (i) the signals generated by the electrodes and (ii) the indication of the spatial deformation provided by the fiber-optic shape sensor, and estimating a position of the distal-end assembly in the body based on the generated signals, the *a-priori* known distances and the indication of the spatial deformation.

6. The method according to claim 5, wherein estimating the position of the distal-end assembly comprises:
estimating position coordinates of the sensing-electrodes using the generated signals;
locally-scaling the position coordinates based on *a-priori* known distances; and
correcting the locally-scaled position coordinates based on the indication of the spatial deformation provided by the fiber-optic shape sensor.
